(19) Europäisches Patentamt
European Patent Office
Office européen des brevets

(11) Publication number: 0 280 051 A1

# (12) EUROPEAN PATENT APPLICATION

(21) Application number: 88101105.0

(22) Date of filing: 26.01.88

(51) Int. Cl.⁴: C07K 5/06, C12P 37/00, C12P 35/00, C12N 9/00, //C12R1/75

(30) Priority: 17.02.87 US 15062

(43) Date of publication of application: 31.08.88 Bulletin 88/35

(84) Designated Contracting States: AT CH DE FR GB IT LI SE

(71) Applicant: QUEEN'S UNIVERSITY AT KINGSTON

Kingston Ontario K7L 3N6(CA)

Applicant: MASSACHUSETTS INSTITUTE OF TECHNOLOGY
77 Massachusetts Avenue
Cambridge, MA 02139(US)

(72) Inventor: Banko, Gerald. Chemie Linz A.G.
CEC-BI St.Peter Strasse 25
A-4026 Linz(AT)
Inventor: Wolfe, Saul
R.R.Number 1
Kingston Ontario K/L 4V1(CA)
Inventor: Demain, Arnold
65 Grove Street, Apt. 251
Wellesley, Massachusetts(US)

(74) Representative: Popp, Eugen, Dr. et al
MEISSNER, BOLTE & PARTNER
Widenmayerstrasse 48 Postfach 86 06 24
D-8000 München 86(DE)

## (54) ACV synthetase.

(57) A process for producing peptides and β-lactam antibiotics from their amino acid precursors, and analogs thereof, is described. A mixture of L-α-aminoadipic acid (A), L-cysteine (C) and L-valine (V) is enzymatically converted into LLD-ACV by a glycerol stabilized cell free extracted of Cephalosporium acremonium (Acremonium chrysogenum ATCC48272). The cell free extract is a single enzyme defined as ACV-synthetase which requires all three L-amino acids for maximum activity.

EP 0 280 051 A1

# ACV SYNTHETASE

## Background of the Invention

1. Field of the Invention

This invention relates to a cell free process for producing peptides and β-lactam antibiotics from the amino acid precursors and analogs thereof. More particularly this invention relates to enzymatic synthesis of δ-( L -α-aminoadipyl)- L -cysteinyl- D -valine (ACV) and analogs thereof for subsequent conversion to a penicillin, cephalosporin or cephamycin.

2. The Prior Art.

The cell free synthesis of penicillin and certain related cephalosporins from ACV tripeptide precursors thereof using an extract from the eukaryotic organism Cephalosporium acremonium is known, and attention is directed to Demain et al U.S. patents 4,178,210 issued 11 December 1979, 4,248,966 issued 3 February 1981, 4,307,192 issued 22 December 1981. The lability of the epimerase agent described in these references is believed to preclude use of cell free extracts of C. acremonium for high yield commercial production of cephalosporins. This belief led Wolfe et al to investigate the production of stable cell free extracts from prokaryotic organisms such as Streptomyces clavuligerus, Streptomyces lipmanii and Streptomyces cattleya, and attention is directed to Wolfe et al U,S. patents 4,510,246 issued 9 April 1985, 4,536,476 issued 20 August 1985, and 4,579,818 issued 1 April 1986, and use of these extracts to produce the desired penicillins and related cephalosporins from the tripeptide precursors. Each of these processes, however, requires the peptide precursor and generally the chemical synthesis thereof is relatively costly.

It is, therefore, an object of the present invention to provide a biosynthetic process for producing ACV and analogs thereof.

## Summary of the Invention

It has now been discovered that appropriately treated extracts of C.acremonium convert a mixture of the three amino acids L -α-aminoadipic acid (A), L -cysteine(C) and L -valine (V) into ACV.

Thus, by one aspect of this invention there is provided a process for providing peptides from the amino acid precursors thereof, comprising: treating a mixture of said amino acids with a disrupted cell preparation of an organism producing at least one of penicillins, cephalosphorins, and cephamycins.

By another aspect there is provided A-C-V synthetase.

By yet another aspect a process for producing peptides from the amino acid precursors thereof, comprising: treating a mixture of said amino acids with a disrupted cell preparation of an organism producing at least one of penicillins cephalosporins and cephamycins.

## Description of the Preferred Embodiments

The biosynthesis of penicillins and cephalosporins is a linear process in both eukaryotic and prokaryotic organisms. The process begins, at the amino acid oxidation level, with the coupling of L -α-aminoadipic acid, L -cysteine and L -valine to form the tripeptide δ-( L -α-aminoadipyl)- L -cysteinyl- D -valine (LLD-ACV). This peptide

(1)

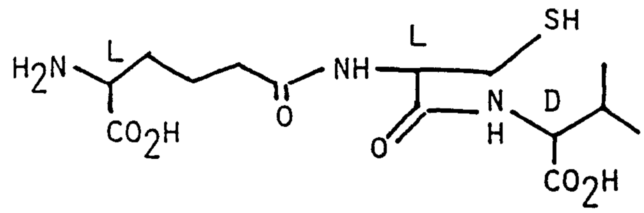

is then converted sequentially into isopenicillin N,

(2)

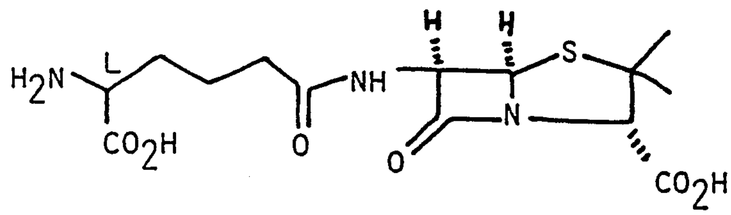

penicillin N,

(3)

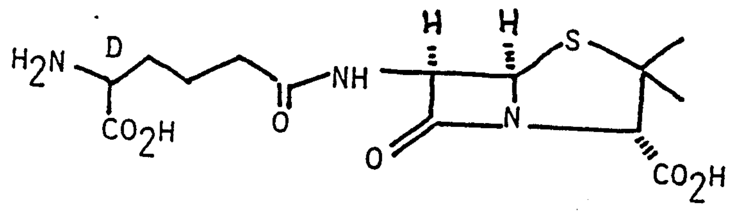

desacetoxycephalosporin C, (4a) X = H,

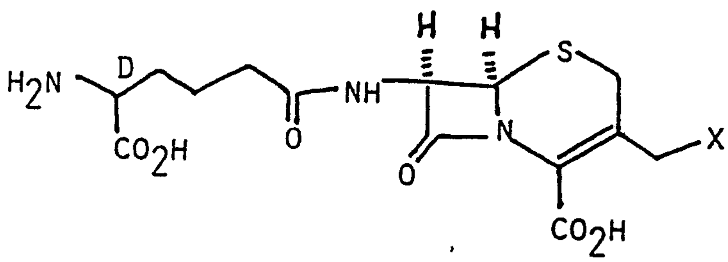

(4)

desacetylcephalosporin C (4b): X = OH, and cephalosporin C (4c): X = OAc or carbamoyloxycephalosporin C (4d): X = $OCONH_2$, the nature and oxidation level of the ultimate product depending upon the organism.

As described in the references above, each of the steps has been observed under cell-free conditions, using homogeneous enzymes, functionally purified enzymes, or mixtures of enzymes, and the sequence 1→2→3→4(a)→4(b) has also been accomplished in quantitative yield on a single immobilized enzyme reactor. A variety of analogs of ACV, in which the L -α-aminoadipyl and/or D -valinyl moieties have been altered by chemical synthesis, are accepted by one or more of the antibiotic-forming enzymes, and converted into nuclear and/or side chain modified penicillins/cephalosporins. Several of these nuclear modified cephalosporins have also been prepared by multi-step chemical syntheses from penicillin precursors. It has thus been demonstrated that novel ring systems, in sufficient quantities for biological evaluation, are more readily accessible from peptide precursors and the appropriate combinations of enzymes and cofactors, than by the more traditional methods of organic chemistry. It must be emphasized, however, that heretofore a peptide precursor has always been required.

Nonetheless, the strategy just described still requires a peptide precursor. Although the requisite chemical syntheses have been solved, and L -carboxymethylcysteine found to be an effective alternative

to L-α-aminoadipic acid, a replacement of the valinyl moiety of the peptide still requires the synthesis, resolution and incorporation of a D-amino acid. In addition, an average of 10-13 protection, coupling and deprotection steps are necessary, from the amino acids, for each new peptide. An enzymatic synthesis of tripeptide analogs of LLD-ACV would, therefore, be of value.

The formation of LLD-ACV from its amino acid precursors has received only sporadic biosynthetic attention. In an early study, a tripeptide was reported to be formed by a cell-free preparation from Penicillium chrysogenum, but the configurations of the amino acids in this peptide were not determined. Experiments with particulate fractions of Cephalosporium sp. suggested that L-α-aminoadipyl-L-cysteine (LL-AC) is an intermediate in the formation of LLD-ACV and that L-to D-epimerization of valine occurs during its attachment to LL-AC. More recently, AC-synthetase activity in cell-free extracts of P. chrysogenum has been confirmed, and soluble extracts of a non-antibiotic producing mutant of Cephalosporium acremonium were found to give 0.1-0.4% incorporation of labelled amino acids into LLD-ACV, and 0.03-0.15% incorporation into LL-AC. It was thought that LLD-ACV biosynthesis parallels glutathione biosynthesis, and involves the action of two separate enzymes.

It has now been found, however, that the ACV-synthetase of C. acremonium C-10 is a single multifunctional enzyme, with broad substrate specificity, whose behavior is more properly compared to that of the multifunctional enzymes associated with the biosyntheses of the peptide antibiotics gramicidin S, tyrocidine, bacitracin, polymyxin and enniatin.

It was first thought that the ATP-and $Mn^{2+}$-or $Mg^{2+}$-dependent activity that is stabilized by addition of glycerol during the preparation of cell-free extracts, was caused by the presence of a barely detectable AC-synthetase. If two enzymes were required for LLD-ACV synthesis, and the first exhibited inhibition by its product, LLD-ACV formation via the reaction LL-AC + L-valine would be faster than LL-AC formation from L-α-aminoadipate + L-cysteine. That this is the case is seen in Table I, which shows data for the two reactions.

TABLE I. Peptide Formation by ACV Synthetase[a]

| Reactants | Product | Conversion ( g/ml) 15 min | 30 min | 60 min |
|---|---|---|---|---|
| A + C | LL-AC | | | 0.2 |
| LL-AC+V | LLD-ACV | 0.9 | 1.1 | |
| A+C+V | LLD-ACV | 5.2 | 10.2 | |

[a] Typical reaction mixtures contained desalted extract (about 0.5 mg protein), 20 µg cycloheximide, 10 mM ATP, 10 mM $MgC1_2$, 5 mM dithiothreitol, 5 mM L-α-aminoadipic acid, l mM L-cysteine and 5 mM L-valine, all components dissolved in 100 µl of 100 mM MOPS/KOH at pH 7.5. Reactions were performed at 25°C in a water bath shaker, and terminated by addition of 25 µl of 20% trichloroacetic acid. Precipitated protein was removed by centrifugation, and the supernatant stored at -20°C prior to analysis. (MOPS = 3-[N-Morpholino] propanesulfonic acid, - a biological buffer available from Sigma Chemical Co.

However, Table I also shows the unexpected finding that the conversion of L-α-aminoadipic acid + L-cysteine + L-valine to LLD-ACV is far more rapid. These observations are not compatible with the hypothesis that two enzymes are required are required for the synthesis of LLD-ACV from its components. Rather, a single enzyme, hereinafter ACV-synthetase, must carry out the synthesis of LLD-ACV, and this enzyme requires binding of all three amino acids for maximum activity.

The stereochemistry of the tripeptide produced in these experiments was established by HPLC comparison with authentic LLD-ACV and also by its isolation from reaction mixtures and conversion to isopenicillin N using isopenicillin N synthetase. The L → D epimerization of valine must occur during tripeptide formation, since D-valine is not accepted as a substrate (Table II),

TABLE II. Formation of ACV Analogs by Single Amino Acid Replacements

| Amino acid replacement [5 mM] | Tripeptide formed [pmoles/mg protein x min] |
|---|---|
| none | 130 |
| for L-α-aminoadipic acid: | |
| L-carboxymethylcysteine | 63.5 |
| L-glutamic acid | 1.9 |
| for L-valine: | |
| L-allo-isoleucine | 24.9 |
| L-α-aminobutyric acid | 8.4 |
| glycine | < 1.0 |
| D-valine | < 1.0 |

and incubation of ACV-synthetase with LLL-ACV in the presence of isopenicillin N synthetase, oxygen, and the cofactors $Fe^{2+}$ and ascorbate does not lead to isopenicillin N.

The optimal concentrations of ATP and $Mg^{2+}$ were found to be 10mM each, and the specific activity of ACV-synthetase was about 20% higher in the presence of 10 mM $Mg^{2+}$ than in the presence of 10 mM $Mn^{2+}$. The apparent Km values of the synthetase for the amino acids are: A, 0.17 mM; C, 0.026 mM and V, 0.34 mM.

The formation of ACV analogs was examined under the same conditions as for LLD -ACV (100 mM MOPS/KOH buffer at pH 7.5, 25°C and shaking at 250 rpm). Table II summarizes the activities observed upon various single amino acid replacements. These reaction mixtures were analyzed by HPLC, and the retention time for each analog was established using the corresponding authentic tripeptide prepared by chemical synthesis. Addition of the authentic tripeptide to a reaction mixture resulted in a single peak in each case. As seen in Table II, peptide analogs of LLD -ACV were obtained by replacement of A with L -carboxymethylcysteine or L -glutamic acid, and by replacement of V with L -allo-isoleucine or L -α-aminobutyric acid. Tripeptide formation was not observed when L -valine was replaced by glycine, or D -valine. When glycine was added as a fourth amino acid, no tetrapeptide was formed and LLD -ACV synthesis was unaffected.

Fructose-1,6-diphosphate, glucose-1-phosphate, orthophosphate, and L -glutamate were found to inhibit ACV-synthetase. L -Methionine, glutathione, L -leucine, L -isoleucine and D -valine (all added at a concentration of 5 mM) had no effect. No component having a positive effect upon the synthetase could be found.

**Example 1.**

Preparation of cells.

Cephalosporium acremonium C-10 (Acremonium chrysogenum ATCC 48272) was maintained on slants and grown in seed medium no. 1 (Glucose 1%, $CaCO_3$ 0.5%, corn steep liquor 3.0%, corn starch 3.0%, pH 7.0). Fermentations were carried out in a medium which contained 3% sucrose, 3.2% soy bean meal, 0.3% DL-methionine and 0.15% $CaCO_3$ (pH 6.8). One hundred ml of medium was used in 500 ml Erlenmeyer flasks. Four ml of the seed culture was used as inoculum for each flask. The fermentations were performed at 20° on a 250 rpm rotary shaker. Mycelia were harvested by centrifugation (1400×g for 10 min) when the cephalosporin titer reached 1000 to 1500 µg per ml.

**Example 2.**

Preparation of cell free extract.

Mycelia from Example 1 were washed twice with cold 50 mM Tris/HC1 buffer (pH 7.2) containing 100 mM KC1. After centrifugation, the pellet was resuspended in a volume of cold 100 mM MOPS/KOH buffer (pH 7.2) equal to twice the pellet volume; the buffer also contained 50 mM KCl, 30 mM 2-mercaptoethanol, 20 mM EDTA and 50% glycerol (v/v). The cells were disrupted in a French Press (Aminco) at 8000 psi and the debris removed by centrifugation (30,000×g, 15 min). The clear yellow supernatant fluid was stored at -20°C or immediately desalted by passage through a Sephadex G-15 column using an elution buffer of 100 mM MOPS/KOH (pH 7.2) containing 50 mM KCl and 20% glycerol (v/v). The resulting solution contained 10 to 20 mg protein per ml and was either used immediately for the cell-free reaction or frozen for future use.

**Example 3.**

Cell free reaction and analysis

Stored frozen extract (from Example 2) was thawed, desalted over Sephadex ®G-15, and eluted with 100 mM MOPS/KOH at pH 7.5. Typical reaction mixtures contained desalted extract (about 0.5 mg protein), 20 µg cycloheximide, 10 mM ATP, 10 mM $MgCl_2$, 5 mM dithiothreitol, 5 mM L-α-aminoadipic acid, 1 mM L-cysteine and 5 mM L-valine, all components dissolved in 100 µl of 100 mM MOPS/KOH at pH 7.5. Reactions were performed at 25°C in a water bath shaker, and terminated by addition of 25 µl of 20% trichloroacetic acid. Precipitated protein was removed by centrifugation, and the supernatant stored at -20°C prior to analysis.

Analyses were performed following derivatization with monobromobimane: to a 20 l sample were added successively 2 µl of 3M KOH, 4 µl of 0.03M dithiothreitol in 2M $NH_4HCO_3$ (pH 8.2), and 4 µl of 0.3M monobromobimane in acetonitrile. This mixture was stored for 15 min. in the dark, and then extracted with methylene chloride (4 × 200 l). Five µl of 1M citric acid (pH 2.2) was added, and 25 µl of this final solution was injected into the HPLC apparatus.

The HPLC analysis employed two solutions, viz., A, 880 mg for pentanesulfonic acid, 2.5 ml of acetic acid in l liter $H_2O$ (pH 3.6); B, 880 mg of pentanesulfonic acid, 2.5 ml acetic acid, 50 ml water and 950 ml acetonitrile. Gradient elution was performed using 8% B in A to 100 % B over 120 min at a flow rate of 1.5 ml/min to obtain the data of Tables 1 and 2 above.

## Claims

1. A process for producing peptides from the amino acid precursors thereof, comprising: treating a mixture of said amino acids with a disrupted cell preparation of an organism producing at least one of penicillins, cephalosporins, and cephamycins.

2. A process as claimed in claim 1 wherein said disrupted cell preparation is treated to yield a cell free extract.

3. A process as claimed in claim 1 wherein said peptide is a tripeptide.

4. A process as claimed in claim 3 wherein said tripeptide is LLD -ACV or an analog thereof.

5. A process as claimed in claim 4 wherein said analogs are selected from L -carboxymethylcysteine, L-glutamic acid, L -allo-isoleucine and L -α-aminobutyric acid.

6. A process as claimed in claim 1 wherein said organism is Cephalosporium acremonium (Acremonium chrysogenum).

7. A process as claimed in claim 4 wherein said cell free extract is glycerol stabilized.

8. A process as claimed in claim 7 wherein said mixture comprises L -α-aminoadipic acid, L -cysteine and L -valine.

9. A process as claimed in claim 8 wherein said LLD -tripeptide is converted to isopenicillin N.

10. A process as claimed in claim 9 wherein said isopenicillin N is converted to penicillin N.

11. A process as claimed in claim 10 wherein said penicillin N is converted to desacetoxycephalosporin C.

12. A process as claimed in claim 11 wherein said desacetoxycephalosporin C is converted to desacetylcephalosporin C.

13. A process as claimed in claim 12 wherein said desacetylcephalosporin C is converted to cephalosporin C or carbamoyloxycephalosporin.

14. A process as claimed in claim 12 wherein said desacetylcephalosporin C is converted to carbamoyloxycephalosporin C.

15. A process as claimed in claim 12 wherein said desacetylcephalosporin C is converted to cephamycins.

16. A-C-V synthetase

17. ACV Synthetase as claimed in claim 16 which is stimulated by ATP and magnesium ($Mg^{2+}$) or manganese ($Mn^{2+}$) co-factors.

18. A process for producing β-lactam antibiotics comprising:

(a) providing a mixture comprising L -α-aminoadipic acid (A), L -cysteine (C) and L -valine or analogs thereof,

(b) reacting said mixture with enzyme derived from a cell free extract of a eukaryotic β-lactam producing organism so as to produce an LLD -tripeptide and,

(c) converting said tripeptide into a selected said β-lactam antibiotic.

European Patent Office

# EUROPEAN SEARCH REPORT

Application Number

EP 88 10 1105

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 174 129 (NATIONAL RESEARCH DEVELOPMENT CO.)<br>* Claims *<br>--- | 1-18 | C 07 K 5/06<br>C 12 P 37/00<br>C 12 P 35/00<br>C 12 N 9/00 //<br>C 12 R 1/75 |
| Y | EP-A-0 144 170 (QUEEN'S UNIVERSITY AT KINGSTON)<br>* Claims *<br>--- | 1-18 | |
| Y | EP-A-0 102 216 (QUEEN'S UNIVERSITY AT KINGSTON)<br>* Claims * & US-A-4 510 246 (Cat. D)<br>--- | 1-18 | |
| D,Y | US-A-4 307 192 (A.L. DEMAIN)<br>* Whole document *<br>----- | 1-18 | |

TECHNICAL FIELDS SEARCHED (Int. Cl.4)

C 07 K 5/00
C 12 P 37/00
C 12 P 35/00
C 12 N 9/00

The present search report has been drawn up for all claims

| Place of search | Date of completion of the search | Examiner |
|---|---|---|
| THE HAGUE | 26-05-1988 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document